# EUROPEAN PATENT APPLICATION

(11) **EP 1 374 954 A1**
(43) Date of publication of application: **02.01.2004**
(21) Application number: 01272241.9
(22) Date of filing: 02.02.2001
(51) Int. Cl.: A63B 69/00, G06F 17/60

(54) **TRAINING SYSTEM**

(30) Priority: 26.12.2000 JP 2000395233
(71) Applicant: MORINAGA & CO., LTD., Tokyo 108-8403 (JP)
(72) Inventor: UDA, Yoshikatsu, Tokyo 146-0092 (JP)
(74) Representative: Brykman, Georges
(86) International application number: PCT/JP2001/000755
(87) International publication number: WO 2002/051507

(57) **Abstract**

The present invention relates to a training system in which the user 100 and the training business 200 are connected via a network such as the Internet, and the user purchases a CD-ROM 101 that stores explanations using video and audio, for example, of the methods of a training program, and carries out the training following the contents of this CD-ROM. In addition, the user's personal information is recorded in advance by the training business, and the training business is notified about the training results data depending on the stage of the progress of the program. The trainer 207 on the business side assesses the state of progress of this user's training from the data that is sent, sends assessment data that includes comments such as advice to the user, and at the same time provides the training program that is to be carried out next.

## Description

### BACKGROUND OF THE INVENTION

### Field of the Invention

The present invention relates to a training system advantageously used for carrying out fitness training via a network.

### Description of the Related Art

Recently, the improvement of health has been vigorously promoted, and many people are undertaking training by various methods outside and inside the home. For example, in the training classes in sports gyms and fitness clubs, aerobics and training that employ various types of equipment are carried out based on the guidance of specialists such as instructors and trainers.

However, in the training classes such as those mentioned above, when people receive instruction, there are variations in the instructors ability to instruct. In addition, it must be said that there are cases in which training is not carried out according to an appropriate program based on objective data depending on the progress of the training, and that the appropriate training is not always efficiently carried out due to the motivation of the student. Thus, there is the problem that the effect of the training does not appear as anticipated, and the student's motivation weakens and dissipates without having reached any targets, and the time and money expended up to this time become wasted.

In addition, there are the problems that in the case that the user does not have training equipment or there is no training facility near his or her home, no training can be carried out, or the user must commute to a distant training facility.

In consideration of the actual situation of conventional fitness training, it is an object of the present invention to provide a training system that allows always carrying out training using an appropriate training program and guiding the user from a distance, and in addition anticipates the development of a new fitness training business.

### SUMMARY OF THE INVENTION

In order to attain the objects described above, the training system according to the present invention comprises a communication device (for example, a network such as the Intemet or the home page 201 in the embodiments) for communication with a user's terminal; a database device (for example, each of the databases 203, 204, 206 in the embodiments) for storing data related to personal information and training transmitted from the user's terminal; a program preparation device (for example, the data analysis part 205 and the trainer terminal 208 in the embodiments) that analyzes training results data sent from the user's terminal by referring to the data in the database, and prepares and sends to the user's terminal the next training program based on the results of this analysis; and a control device (for example, the control system 202 in the embodiments) for controlling each of the devices.

Therefore, according to the present invention, each time the user executes a program the training results data that shows the results of the training are sent, and based thereon a program preparation device prepares the next training program and sends it to the user, and thereby the user can always execute training using an appropriate training program prepared in conformity to the condition of progress of the training, and the targets can be reliably achieved.

In addition, in the above-described training system, the user's terminal uses a recording medium that stores information explaining the method of executing the training program, and the user carries out the training by selecting content according to the prepared training program from this recording medium.

The user carries out training using a recording medium such as a CD-ROM on with the method of executed the training program is recorded, and thus the user can easily understand the method of the training by watching the images played on the recording medium. Thereby, the training can be correctly carried out, and the targets can be reliably approached. In addition, a business can earn a profit by selling the recording medium. Generally, by providing the user in advance the image information that generally requires a large amount of information on a high capacity medium such as a CD-ROM or DVD-ROM, the amount of information required for sending the program information is decreased, and in addition to reducing the transmission time, the quantity of communication can be limited.

In addition, in the above-described training system, the program preparation device sends a control signal that allows reading only a predetermined recorded part of the recording medium when the prepared training program is sent to the user's terminal.

By allowing only the recorded part of the recording medium related to the relevant training program to be read, the motivation of the user can be strengthened, and the targets reliably accomplished.

In addition, in the above-described training system, the program preparation device can determine that the training level should be increased based on said training results data, and prepare the next training program depending on the result of this determination.

In this manner, by determining that the training level should be increased based on said training results data, and preparing the next training program depending on the result of this determination, training according to a program that conforms to the state of progress of the training can be realized.

Furthermore, in the above-described training system, the program preparation device prepares assessment data that includes comments to the user based on the training results data and sends the assessment data to the user.

In this manner, by the program preparation device preparing assessment data that includes comments to the user based on the training results data and sending the assessment data to the user, the user can continue training while correctly understanding the state of progress of his or her training.

Furthermore, in the above-described training system, an accounting system for assessing the user a fee at predetermined times during the training stages can be provided.

In this manner, by providing an accounting system for assessing the user a fee at predetermined times during the training stages, a fitness training business can be realized.

Furthermore, in the above-described training system, the program preparation device can determine from the training results data whether or not the user has continuously carried out the same training program a predetermined number of times, and in the case that the user has carried out the training program a predetermined number of times, can recommend a program having another category as the next training program.

In this manner, by recommending a program having another category as the next training program in the case that the user has continuously carried out the same training program a predetermined number of times, a training system in which that the user does not lose interest can be realized.

In addition, in the above-described training system, the program preparation device prepares and sends to the user's terminal a first training program based on said personal information.

According to the training system configured in this manner, a first training program for the user can be prepared based on the user's personal information.

### BRIEF DESCRIPTION OF THE DRAWINGS

Fig. 1 is a structural drawing showing the training system according to the embodiment of the present invention.
Fig. 2 is a flowchart showing the training sequence of the training system according to the embodiment of the present invention.
Fig. 3 is a structural drawing showing an example of the training program in which resistance training is used as an example.
Fig. 4 is a structural drawing showing an example of a part of the training result data.
Fig. 5 is a flowchart showing the processing of level determination.

### DETAILED DESCRIPTION OF THE INVENTION

Below, an embodiment of the present invention will be explained referring to the drawings.

In the training system according to the present embodiment, the user and a training business are connected via a network such as the Internet, and the user purchases a CD-ROM on which has the explanations of the methods of the training program provided by images and voice are recorded. The user carries out training according to the contents of this CD-ROM, and at the time of the purchase, the user's personal information is registered in advance by the training business. In addition, depending on the stage of advancement of the program, data that represents the results of the training are reported to the training business. The trainer on the business side assesses the state of progress of the training of this user from the data that is sent, and sends to the user assessment data that includes comments such as advice. At the same time, the user is instructed about the program on the CD-ROM that should be carried out next. In this manner, the training can be reliably carried out at stages following a predetermined course.

Next, the present embodiment will be explained concretely.

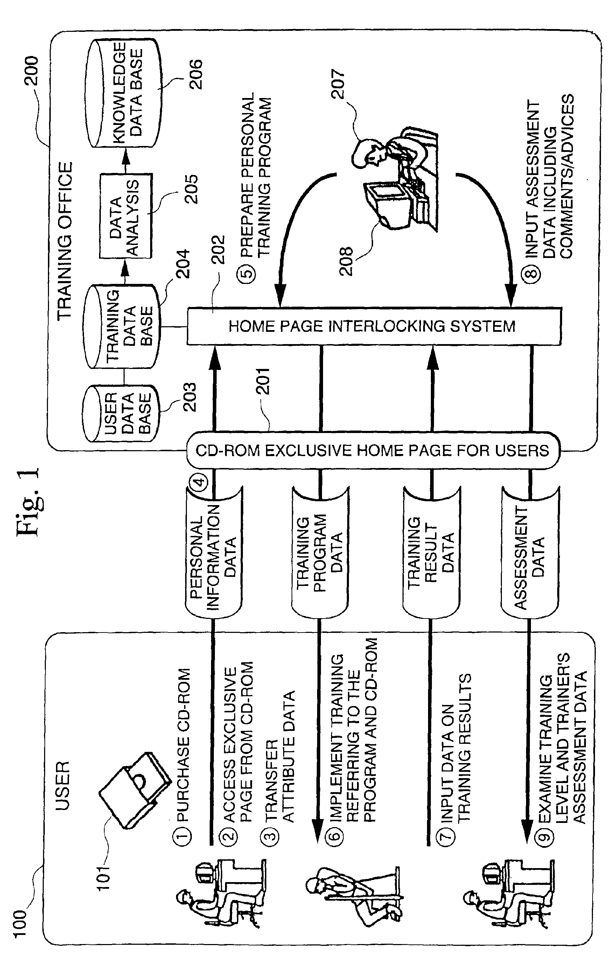

Fig. 1 is a structural drawing showing schematically the training system according to the present embodiment.

In Fig. 1, the present system comprises a user 100 and a training business 200 (hereinbelow, referred to simply as a "business") that serves as a training center, and the user 100 and the business 200 are connected by a communication network such as the Internet. Moreover, in the following explanation, depending on the case, the user denotes the person who carries out the training, a user terminal that is owned by the user, or a user terminal that is placed in the training class that the user attends.

The user terminal is operated by the user who carries out the training or the specialist operator in the training class. In addition, the training system may be structured by a plurality of user terminals disposed regionally or nationally, or two or more business 200 can serve as training centers to comprehensively manage this plurality of user terminal via the network.

The user 100 first purchases the CD-ROM 101. On this CD-ROM 101, content is recorded that explains the ways and methods for carrying out the training program s in the various training programs using characters, audio, or videos. At the business 200, there is a home page dedicated to the CD-ROM user on a Web site of a server, and at the same time, a control system 202 that communicates with the home page 201, a customer DB (database) 203, a training DB 204, a data analyzing part 205, a knowledge DB 206, and the trainer terminal 208 that the trainer 207 uses, and the like are provided.

Next, an outline of the training sequence according to the present system will be explained in the order from ① to ⑨ in Fig. 1.
① First, the user 100 purchases the CD-ROM 101.
② The user accesses the home page 201 via the network using this CD-ROM.
③ After connecting to the home page and carrying out user authentication, the user inputs personal data. The personal data includes, for example, the address and name, age, training targets, height, weight, present health status, or the like.
④ The business 200 receives this data via the control system 202 from the home page, and registers it in the customer DB 203.
⑤ Next, the trainer 207 selects a program that matches the user based on the above-described personal information and the information in each of the databases using the trainer terminal 208, prepares a training program, and sends it to the user.
⑥ The user carries out the training according to the contents of the CD-ROM corresponding to the program that has been sent.
⑦ When the user finishes a predetermined program, he or she inputs the training results data.
⑧ The trainer determines whether the user should progress to the next stage based on the training results data that has been sent, prepares comments that include for example the assessment results and advice and the assessment data, and sends them to the user.
⑨ The user refers to the assessment data.

Subsequently, the sequence ② through ⑨ is repeated at each stage of the training, the user refers to the training program and the assessment data, and carries out training progressing through stages in sequence. At the end of each stage, the training results data is sent and stored in the training DB 204. In addition, the data analyzer part 205 refers to the customer DB 203 and the training DB 204 based on the instructions of the trainer, and at the same time, referring to the knowledge data related to the training stored in the knowledge DB 206, the data from the user is analyzed and the above-mentioned assessment results and assessment data that includes for example comments and advice are prepared, and the next training program is prepared. The next training program can be a program that is a higher level than the previous program, a program that is the same level as the previous program, or a program of another category.

Moreover, when the training program is sent to the user, only the content of the CD-ROM corresponding to this program is opened, while other unrelated parts of the program cannot be opened. In this manner, the user's motivation to attain targets while progressing through the stages is stimulated, and can be reliably encouraged to make efforts to approach targets.

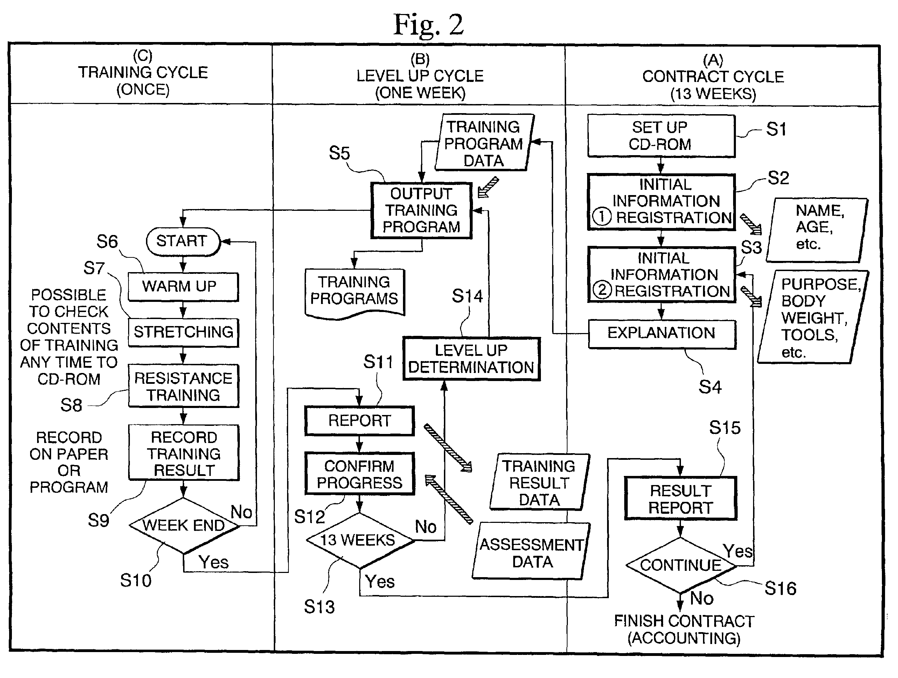

Next, a more concrete embodiment of the training sequence described above will be explained in detail referring to the flowchart in Fig. 2.

Fig. 2 comprises (A) a contract cycle for training (here, one cycle lasts 13 weeks), (B) a level improvement cycle (one stage takes one week), and (C) one training cycle (one training program is one cycle, and normally two cycles per week are carried out). The process indicated by the bold line in the figure denotes the data transfer processing. This transfer processing includes not only on-line data processing, but also data transfer by batch processing where the training business serves as an intermediary. Moreover, the training in one program normally lasts three months (12 weeks), and here the first one week comprises pre-conditioning training.

As types of training, there are, for example, aerobics, stretching, speed training, plyometrics, and resistance training, and as well as training for athletes, fitness improvement, children and the elderly. Here, the case of resistance exercises in which the body strength is increased by applying a load to the body by barbells, dumbbells, exercise machines, dead weight, calisthenics, or the like is explained.

In Fig. 2, first the user sets up the CD-ROM in the user terminal (step S1, hereinbelow, "step" is omitted), and as initial information, the personal information is input, and registration steps ① and ② are carried out (S2, S3). Moreover, when the CD-ROM is purchased, a password can be assigned, and at registration step ① the password is entered and authentication carried out. In addition, at registration step ①, the user registers his or her name, age, or the like, and at registration step ②, the data related to the training targets, weight, equipment, health condition, or the like is input.

Next, there is an explanation of the first step of the training on the CD-ROM (S4), and in addition, the initial training program data is sent from the business (S5), and is displayed on the user's terminal.

Next, the training begins. According to the program that has been sent, the training begins with warm-up exercises (S6), the subsequently training comprising stretching (S7) and resistance training (S8) is carried out as one cycle. When the user completes one cycle, the results of this training are recorded as training result data according to a predetermined format (S9).

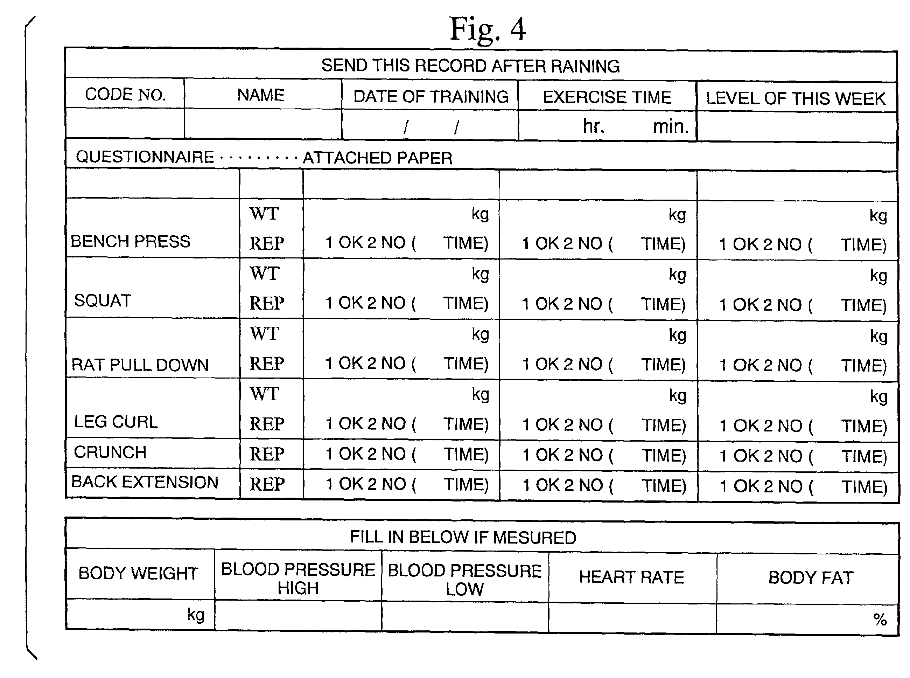

As the content of the training results data, there is numerical data for each category of training (explained below in the description of Fig. 4), and in addition, there is the data in a prescribed questionnaire format. Question topics for the questionnaire could include, for example, whether the present training is too unchallenging, too strenuous, or appropriate, or the number of days in a week that the training was continued or missed.

Each set of training results data is reported to the business during the weekend (S10, S11), where, for example, two cycles are carried out in one week and the sequence of S5 to S9 described above comprises one cycle. Next, assessment data is prepared by referring to each DB based on the reported training result data by the data analyzer based on the instructions of the trainer at the business. In addition, question topics in a separate questionnaire based on the present training results data are sent to the user along with the assessment data.

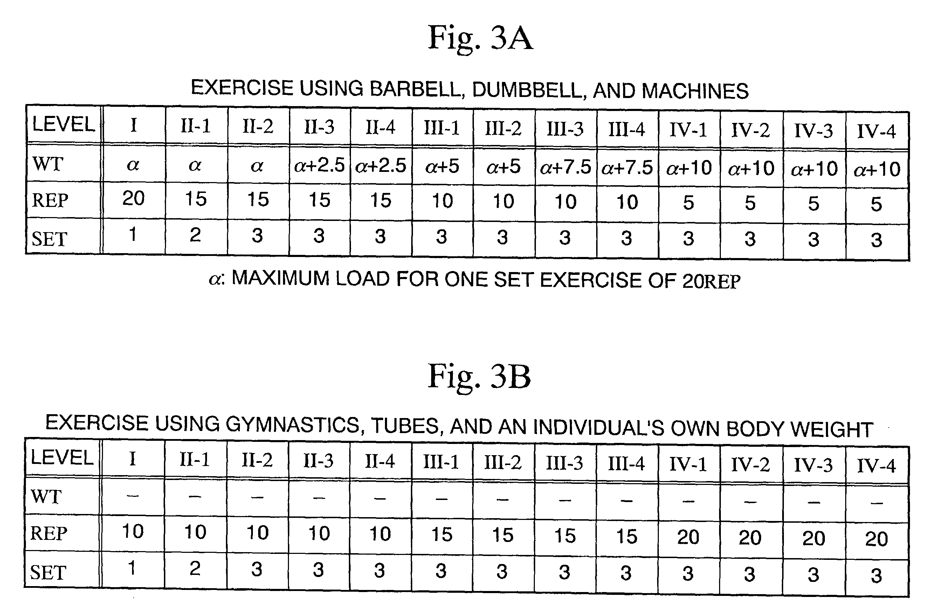

Whether or not the sequence of S4 to S12 described above has been carried out to the end of the contract period unit (for example, 13 weeks) is checked (S21), and if 13 weeks has not elapsed, the training results for one week are examined, and it is determined using as a general determination criteria whether or not the training program in Fig. 3 (described below) was accomplished (S13, No, S21). In addition, if the improvement in level is recognized, a training program for carrying out the next step in the training is prepared and sent to the user (S5). At this time, only pages of the CD-ROM related to the relevant program are selected and a control signal that allows them to be read can be sent to the user.

In addition, based on the new training program, S6 to S10 and S11 to S14, and S4 are carried out. Moreover, at S14, in the case that the improvement in level is not recognized, either the same program is carried out again or the training in another category is carried out.

In S13, when 13 weeks have elapsed, the results of this training are summarized and stored in the DB, and at the same time, reported to the user (S2). In addition, the user is asked whether he or she will continue to carry out different training or the same training, and in the case the user wishes to continue, the procedure returns to S3, and the registration of initial information related to the training is carried out. In the case that the user does not wish to continue, the contract is ended. When the contract has ended, assessment of the user's fees can be carried out. The assessment of fees can be carried out by charging a predetermined fee to the user's account at a financial institution such as a bank.

Moreover, different types of methods for assessing fees can be considered in addition to that described above. For example, fees can be assessed each time a new training program is sent, or the fees can be assessed at an appropriate timing while the training is in progress. In addition, depending on the case, a part of or all of the sales price of the CD-ROM can be included. Furthermore, in the predetermined stages, when the training for a predetermined period is not carried out, fees up until that point can be charged. In addition, according to the results of this fee assessment, the program preparation device can determine the permission to read the predetermined contents in the user's terminal, and can permit reading of the contents after transmitting to the user terminal a signal that allows reading of the relevant contents.

Next, the method of determining the level of improvement will be explained. Figs. 3A and 3B show examples of the training programs for resistance training. Here, the load for training in each level of 13 stages is shown. Fig. 3A shows the case in which barbells, exercise machines, and dumbbells are used, and Fig. 3B shows the case in which dead weight, calisthenics, and flexible tubes are used. WT denotes the weight of the load, REP denotes the number of repetitions, SET denotes the number of sets, and α denotes the initial value of the weight. For example, in the level shown in Fig. 3A, carrying out one set is shown, where lifting a barbell 20 times comprises 1 set. Subsequently, the WT, REP, and SET increase as the level increases.

For the provided program, the user records whether or not the target was attained item by item for each WT, REP, and SET, and reports answers to a questionnaire along with the training result data. The trainer at the business prepares assessment data that indicates the state of progress with respect to this report, and notifies the user about this using, for example, a graph display. The assessment data incorporates comments that include, for example, advice from the trainer. The comments can be prepared automatically depending on the state of the progress. In addition, the initial values of α, β, and γ can be set by the user. Setting criterion could be, for example, whether the program can be performed without assistance or whether the user ran out of breath during the exercise.

Fig. 4 shows an example of the numerical data that the user records for each category in the training result data. As shown in the figure, the values of WT, REP, and SET that the user actually carried out for each category such as for the bench press, squats, lateral pull-downs, or the like, and the measured values for the weight, blood pressure, or the like are recorded.

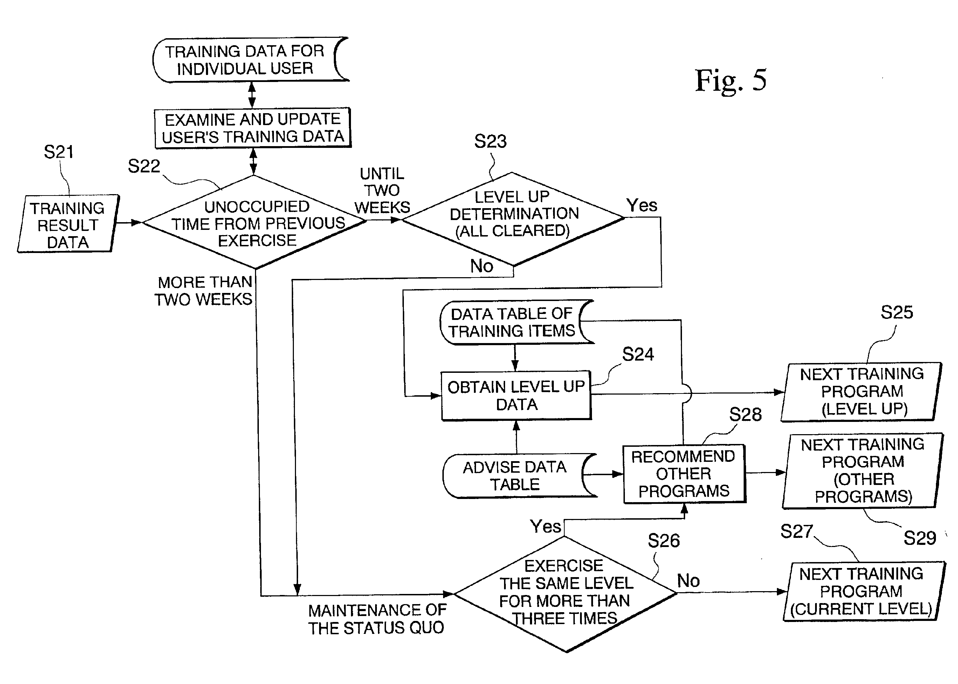

Next, the method of assessing the improvement in the level will be explained referring to Fig. 5. When the user inputs the training result data (S21), it is determined by referring to the customer training data of the DB whether or not two or more weeks have passed since the completion of the user's previous training (S22). At this time, the DB is updated according to the training result data. If two weeks have not passed since the completion of the previous training, an improvement in the level is determined. This determination generally is carried out depending on whether or not the entire training program has been accomplished (refer to Fig. 3) and referring to the questionnaire results (S23).

When an improvement in the level is recognized, data for the improvement in level is obtained by referring to the data tables and the advice data tables for each category in the DB (S24), the next training program is prepared and sent to the user along with the assessment data (S25).

In addition, at S22, in the case that the user has missed training for two or more weeks, to maintain the present condition, next it is determined whether or not the program at the same level has been carried out three times or more (S26). If the program has not been carried out three times or more, the next training program for maintaining the present level is prepared and sent to the user (S27). In the case that the program has been carried out more than three times, it is determined to recommend training from a different category referring to the data tables and advice data tables or the like for the above-described categories (S28). The next training program is prepared according to a different category and sent to the user (S29). This processing by S28 and S29 anticipates that the user will become bored continuing the same menu three times or more, and thus recommends another category.

Moreover, the training system according to the present invention is not limited by the embodiment explained above, and the method of producing the training program and the method of determining the improvement in level can be modified in various manners.

As explained above, according to the training system of the present invention, the next training program is prepared based on the training result data of the user, and thus the training can always be effectively carried out using an appropriate program based on objective data according to the progress of the training. In addition, appropriate guidance can be carried out for the distant user. Therefore, the present invention can be used in a training system in which fitness training is carried out via a network.

## Claims

1. A training system comprising:
a communication device for communicating with a user's terminal;
a database device for storing data related to personal information and training transmitted from the user's terminal;
a program preparation device that analyzes training results data sent from the user's terminal by referring to the data in said database, and prepares and sends to the user's terminal the next training program based on the results of this analysis; and
a control device for controlling each of said devices.

2. A training system according to claim 1, wherein said user's terminal uses a recording medium that stores information explaining the method of executing said training program, and said user selects content according to said prepared training program from this recording medium.

3. A training system according to claim 2, wherein said program preparation device sends a control signal that can read only a predetermined recorded part of said recording medium when said prepared training program is sent to the user's terminal.

4. A training system according to claim 1, wherein said program preparation device determines that the training level should be increased based on said training results data, and prepares the next training program depending on the result of this determination.

5. A training system according to claim 1, wherein said program preparation device prepares assessment data that includes comments to the user based on said training results data and sends said assessment data to the user.

6. A training system according to claim 1, providing an accounting system for assessing the user a fee at predetermined times during the training stages.

7. A training system according to claim 1, wherein said program preparation device determines from the training results data whether or not the user has continuously carried out the same training program a predetermined number of times, and in the case that the user has carried out the training program a predetermined number of times, recommends a program in another category as the next training program.

8. A training system according to claim 1, wherein said program preparation device prepares and sends to the user's terminal a first training program based on said personal information.
